# EUROPÄISCHE PATENTANMELDUNG

(11) **EP 2 072 497 A1**
(43) Veröffentlichungstag der Anmeldung: **24.06.2009**
(21) Anmeldenummer: 07150357.7
(22) Anmeldetag: 21.12.2007
(51) Int. Cl.: C07C 227/16, C07C 229/30

(54) **Verfahren zum Herstellen von 2-Fluoracyl-3-amino-acrylsäure-Derivaten**

(71) Anmelder: Bayer CropScience AG, 40789 Monheim (DE)
(72) Erfinder: Die Erfindernennung liegt noch nicht vor

(57) **Zusammenfassung**

Die vorliegende Erfindung betrifft ein Verfahren zum Herstellen von 2-Fluoracyl-3-aminoacrylsäure-Derivaten durch Umsetzung von fluorierten Carbonsäuren mit Dialkylaminoacrylsäure-Derivaten und Säurehalogeniden.

## Beschreibung

Die vorliegende Erfindung betrifft ein Verfahren zum Herstellen von 2-Fluoracyl-3-amino-acrylsäure-Derivaten durch Umsetzung von fluorierten Carbonsäuren mit Dialkylaminoacrylsäure-Derivaten und Säurehalogeniden. 2-Fluoracyl-3-amino-acrylsäure-Derivate wie z.B. 2-Trifluormethyl-3-aminoacrylsäureester und 2-Difluormethyl-3-aminoacrylsäureester sind wertvolle Zwischenprodukte zur Herstellung von substituierten Pyrazolen, die als Fungizide Verwendung finden können.

Es ist bereits bekannt, dass man trihalogenacylierte Aminoacrylsäureester erhält, wenn man die entsprechenden Chloracroleine mit einem substituierten Amin umsetzt (vgl. Tetrahedron Lett. 1996, 37, 8751 - 8754). Nachteilig an diesem Verfahren ist jedoch, dass die als Ausgangsverbindungen eingesetzten Chloracroleine schwierig herzustellen und für eine technische Anwendung zu teuer sind.

Aus EP-A-1 000 926 ist bekannt, dass man Trihaloacyl-aminopropenoate erhält, indem man Trihalogenacetoacetate mit Dialkylformamidacetalen umsetzt. Nachteilig ist hier, dass als Nebenprodukt die deacylierten Verbindungen auftreten und vom gewünschten Produkt abgetrennt werden müssen.

WO-A-03/051820 offenbar, dass man 2-Perhalogenacyl-3-amino-acrylsäure-Derivate erhalten kann, indem man 3-Aminoacrylsäureester mit Perhalogenallcylcarbonsäureanhydriden umsetzt. Nachteilig ist hier, dass man zur Herstellung der fluorierte Derivate niedrig siedende und teure Säurechloride bzw. Anhydride benötigt. Beim Einsatz von z.B. Trifluoressigsäureanhydrid verliert man ein Äquivalent des wertvollen fluorierten Bausteins.

WO-A-05/042468 lehrt ein Verfahren zum Herstellen von 2-Dihalogenacyl-3-amino-acrylsäureestern bei dem Säurehalogenide eingesetzt werden. Diese Säurechloride sind aufwendig aus den Säuren herzustellen. Außerdem ist ihr Einsatz aufgrund der niedrigen Siedepunkte schwierig.

Die Aufgabe der vorliegenden Erfindung bestand in der Bereitstellung neuer, wirtschaftlicher Verfahren zum Herstellen von 2-Fluoracyl-3-amino-acrylsäure-Derivaten ausgehend von fluorierten Carbonsäuren, die die zuvor beschriebenen Nachteile nicht aufweisen.

Die Aufgabe wurde gemäß der vorliegenden Erfindung gelöst durch ein Verfahren zum Herstellen von 2-Fluoracyl-3-amino-acrylsäure-Derivaten der allgemeinen Formel (I) 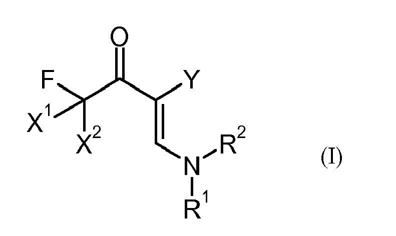 in welcher
- R¹ und: R² unabhängig voneinander ausgewählt ist aus C₁₋₁₂-Alkylresten, C₅₋₁₈-Aryl-, C₇₋₁₉-Alkylaryl-oder C₇₋₁₉-Arylalkyresten, oder
- R¹ und: R² zusammen mit dem Stickstoffatom, an das sie gebunden sind, einen 5- bis 6-gliedrigen Ring bilden können, der gegebenenfalls ein oder zwei weitere Hetero-Atome, ausgewählt aus O, S und einer SO₂-Gruppe, enthalten kann;
- Y: ausgewählt ist aus (C=O)OR³, CN und (C=O)NR⁴R⁵, wobei R³ ,R⁴ und R⁵ unabhängig von einander ausgewählt sind aus C₁₋₁₂-Alkylresten, C₅₋₁₈-Aryl-, C₇₋₁₉-Alkylaryl- oder C₇₋₁₉-Arylalkyresten und
- X¹ und: X² unabhängig voneinander für Fluor, Chlor, Brom, Wasserstoff, C₁₋₁₂-Alkylreste, C₁₋₁₂-Haloalkylreste, C₅₋₁₈-Aryl-, C₇₋₁₉-Alkylaryl- oder C₇₋₁₉-Arylalkyreste stehen,
dadurch gekennzeichnet, dass man fluorierte Carbonsäuren der Formel (II) 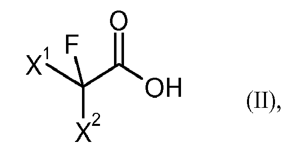 in welcher X¹ und X² die oben genannte Bedeutungen haben, mit 3-Amino-acrylsäure-Derivaten der Formel (III) 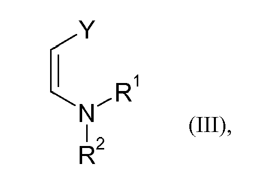 in welcher R¹, R² und Y die oben genannte Bedeutungen haben, in Gegenwart einer Base, mit einem Säurehalogenid der Formel (IV) 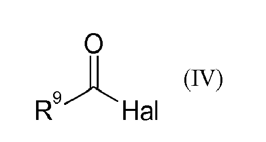 in welcher R⁹ für C₁₋₁₂-Alkylreste, C₃₋₈-Cycloalkylreste, C₁₋₁₂-Haloalkylreste C₅₋₁₈-Aryl- oder C₇₋₁₉-Arylalkyreste und Hal für Halogen steht, umsetzt.

Überraschenderweise lassen sich die 2-Fluoracyl-3-amino-acrylsäure-Derivate der Formel (I) unter den erfindungsgemäßen Bedingungen mit guten Ausbeuten in hoher Reinheit herstellen, womit das erfindungsgemäße Verfahren die im Zusammenhang mit dem Stand der Technik beschriebenen Nachteile nicht aufweist.

### Das erfindungsgemäße Verfahren kann anhand des folgenden Schema (I) erläutert werden :

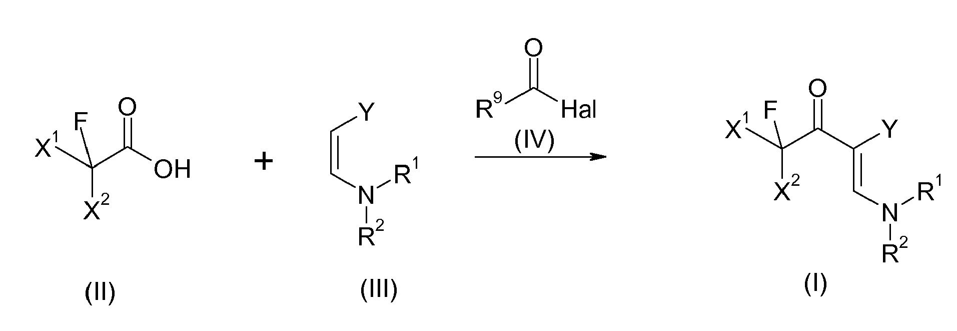

Allgemeine Definitionen Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.

Gegebenenfalls substituierte Gruppen können einfach oder mehrfach substituiert sein, wobei bei Mehrfachsubstitutionen die Substituenten gleich oder verschieden sein können.

Mit einem oder mehreren Halogenatomen (-X) substituierte Alkyl-Gruppen sind beispielsweise ausgewählt aus Trifluormethyl (CF₃), Difluormethyl (CHF₂), CF₃CH₂, ClCH₂, CF₃CCl₂.

Alkyl-Gruppen sind im Zusammenhang mit der vorliegenden Erfindung, soweit nicht abweichend definiert, lineare, verzweigte oder ringförmige gesättigte Kohlenwasserstoff-Gruppen.

Die Definition C₁-C₁₂-Alkyl umfasst den größten hierin definierten Bereich für einen Alkyl-Gruppe. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Alkenyl-Gruppen sind im Zusammenhang mit der vorliegenden Erfindung, soweit nicht abweichend definiert, lineare, verzweigte oder ringförmige Kohlenwasserstoff-Gruppen, die wenigstens eine einfache Unsättigung (Doppelbindung) enthalten.

Die Definition C₂-C₁₂-Alkenyl umfasst den größten hierin definierten Bereich für einen AlkenylGruppe. Im Einzelnen umfasst diese Definition beispielsweise die Bedeutungen Vinyl; Allyl (2-Propenyl), Isopropenyl (1-Methylethenyl); But-1-enyl (Crotyl), But-2-enyl, But-3-enyl; Hex-1-enyl, Hex-2-enyl, Hex-3-enyl, Hex-4-enyl, Hex-5-enyl; Hept-1-enyl, Hept-2-enyl, Hept-3-enyl, Hept-4-enyl, Hept-5-enyl, Hept-6-enyl; Oct-1-enyl, Oct-2-enyl, Oct-3-enyl, Oct-4-enyl, Oct-5-enyl, Oct-6-enyl, Oct-7-enyl; Non-1-enyl, Non-2-enyl, Non-3-enyl, Non-4-enyl, Non-5-enyl, Non-6-enyl, Non-7-enyl, Non-8-enyl; Dec-1-enyl, Dec-2-enyl, Dec-3-enyl, Dec-4-enyl, Dec-5-enyl, Dec-6-enyl, Dec-7-enyl, Dec-8-enyl, Dec-9-enyl; Undec-1-enyl, Undec-2-enyl, Undec-3-enyl, Undec-4-enyl, Undec-5-enyl, Undec-6-enyl, Undec-7-enyl, Undec-8-enyl, Undec-9-enyl, Undec-10-enyl; Dodec-1-enyl, Dodec-2-enyl, Dodec-3-enyl, Dodec-4-enyl, Dodec-5-enyl, Dodec-6-enyl, Dodec-7-enyl, Dodec-8-enyl, Dodec-9-enyl, Dodec-10-enyl, Dodec-11-enyl; Buta-1,3-dienyl, Penta-1,3-dienyl.

Alkinyl-Gruppen sind im Zusammenhang mit der vorliegenden Erfindung, soweit nicht abweichend definiert, lineare, verzweigte oder ringförmige Kohlenwasserstoff-Gruppen, die wenigstens eine zweifache Unsättigung (Dreifachbindung) enthalten.

Die Definition C₂-C₁₂-Alkinyl umfasst den größten hierin definierten Bereich für einen Alkinyl-Gruppe. Im Einzelnen umfasst diese Definition beispielsweise die Bedeutungen Ethinyl (Acetylenyl); Prop-1-inyl und Prop-2-inyl.

Aryl-Gruppen sind im Zusammenhang mit der vorliegenden Erfindung, soweit nicht abweichend definiert, aromatische Kohlenwasserstoff-Gruppen, die ein, zwei oder mehrere Heteroatome, die ausgewählt sind aus O, N, P und S, aufweisen können.

Die Definition C₅₋₁₈-Aryl umfasst den größten hierin definierten Bereich für eine Aryl-Gruppe mit 5 bis 18 Gerüst-Atomen, wobei die C-Atome gegen Heteroatome ausgetauscht sein können. Im Einzelnen umfasst diese Definition beispielsweise die Bedeutungen Cyclopentadienyl, Phenyl, Cycloheptatrienyl, Cyclooctatetraenyl, Naphthyl und Anthracenyl; 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5- Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl; 1-Pyrrolyl, 1-Pyrazolyl, 1,2,4-Triazol-1-yl, 1-Imidazolyl, 1,2,3-Triazol-1-yl, 1,3,4-Triazol-1-yl; 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

Arylalkyl-Gruppen (Aralkyl-Gruppen) sind im Zusammenhang mit der vorliegenden Erfindung, soweit nicht abweichend definiert, durch Aryl-Gruppen substituierte Alkyl-Gruppen, die eine C₁₋₈-Alkylenkette aufweisen können und im Arylgerüst ein oder mehrere Heteroatome, die ausgewählt sind aus O, N, P und S, aufweisen können.

Die Definition C₇₋₁₉-Aralkyl-Gruppe umfasst den größten hierin definierten Bereich für einen Arylalkyl-Gruppe mit insgesamt 7 bis 19 Atomen in Gerüst und Alkylenkette. Im Einzelnen umfasst diese Definition beispielsweise die Bedeutungen Benzyl- und Phenylethyl-.

Alkylaryl-Gruppen (Alkaryl-Gruppen) sind im Zusammenhang mit der vorliegenden Erfindung, soweit nicht abweichend definiert, durch Alkyl-Gruppen substituierte Aryl-Gruppen, die eine C₁₋₈-Alkylenkette aufweisen können und im Arylgerüst ein oder mehrere Heteroatome, die ausgewählt sind aus O, N, P und S, aufweisen können.

Die Definition C₇₋₁₉-Alkylaryl-Gruppe umfasst den größten hierin definierten Bereich für einen Alkylaryl-Gruppe mit insgesamt 7 bis 19 Atomen in Gerüst und Alkylenkette. Im Einzelnen umfasst diese Definition beispielsweise die Bedeutungen Tolyl-, 2,3-, 2,4-, 2,5-, 2,6-, 3,4- oder 3,5-Dimethylphenyl.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart und beansprucht.

### Dialkylaminoacrylsäure-Derivate (III)

Die bei der Durchführung des erfindungsgemäßen Verfahrens als Ausgangsstoffe verwendeten Dialkylaminoacrylsäure-Derivate sind durch die Formel (III) allgemein definiert. 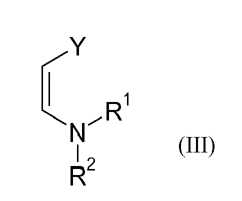 In dieser Formel können
- R¹ und: R² unabhängig voneinander ausgewählt sein aus C₁₋₁₂-Alkylresten, C₅₋₁₈-Aryl-, C₇₋₁₉-Alkylaryl- oder C₇₋₁₉-Arylalkyresten, oder
- R¹ und: R² zusammen mit dem Stickstoffatom, an das sie gebunden sind, einen 5- bis 6-gliedrigen Ring bilden, der gegebenenfalls ein oder zwei weitere Hetero-Atome, ausgewählt aus O, S und einer SO₂-Gruppe enthalten kann,
- Y: ausgewählt sein aus Carbonsäureestergruppen ((C=O)OR³), Nitrilgruppen (CN) und Amidgruppen ((C=O)NR⁴R⁵), wobei R³ ,R⁴ und R⁵ unabhängig von einander ausgewählt sind aus C₁₋₁₂-Alkylresten, C₅₋₁₈-Aryl-, C₇₋₁₉-Alkylaryl- oder C₇₋₁₉-Arylalkylresten.
Vorzugsweise können
- R¹ und: R² unabhängig voneinander ausgewählt sein aus Methyl, Ethyl, n-Propyl oder iso-Propyl,
- R¹ und: R² zusammen mit dem Stickstoffatom, an das sie gebunden sind, einen Piperidinyl-, Pyrolidinyl- oder Morpholidinyl-Ring bilden,
- Y: ausgewählt sein aus (C=O)OR³ wobei R³ ausgewählt ist aus Methyl, Ethyl, n-Propyl oder iso-Propyl.
Besonders bevorzugt können
- R¹ und: R² Methyl sein und
- Y: -(C=O)OC₂H₅ sein.

Beispiele für erfindungsgemäß geeignete Dialkylaminoacrylsäureester sind 3-(N,N-Dimethylamino)-acrylsäuremethylester, 3-(N,N-Dimethylamino)-acrylsäureethylester, 3-(N,N-Diethylamino)-acrylsäureethylester, 3-(N,N-Dimethylamino)-acrylsäurenitril, 3-(N,N-Dimethylamino)-acrylsäuredimethylamid und 3-(N,N-Dimethylamino)-acrylsäurediethylamid wobei 3-(N,N-Diethylamino)-acrylsäureethylester besonders bevorzugt ist.

Verfahren zur Herstellung von Dialkylaminoacrylsäureestem sind im Stand der Technik, beispielsweise in EP-A-0 608 725, vorbeschrieben.

Verfahren zur Herstellung von Dialkylaminoacrylnitrilen sind im Stand der Technik, beispielsweise von Rene et al in Synthesis (1986), (5), 419-420 beschrieben.

Die Dialkylaminoacrylsäure-Derivate können, falls notwendig, beispielsweise destillativ gereinigt werden. Dies ist im Allgemeinen jedoch im Zusammenhang mit der erfindungsgemäßen Reaktion nicht erforderlich.

Das molare Verhältnis von Dialkylaminoacrylsäure-Derivaten zu eingesetzten fluorierten Carbonsäure kann beispielsweise 0,5 bis 3 betragen, bevorzugt 0,8 bis 2, besonders bevorzugt 1,0 bis 1,5.

### Fluorierte Carbonsäuren (II)

Die gemäß der vorliegenden Erfindung verwendeten fluorierte Carbonsäuren sind Verbindungen der allgemeinen Formel (II) 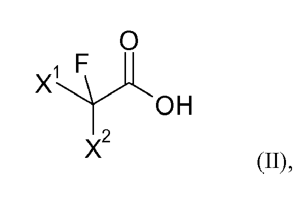 in welcher
- X¹ und: X² unabhängig voneinander für Fluor, Chlor, Brom, Wasserstoff, C₁₋₁₂-Alkylreste, C₁₋₁₂-Haloalkylreste, C₅₋₁₈-Aryl-, C₇₋₁₉-Alkylaryl- oder C₇₋₁₉-Arylalkyreste, vorzugsweise für Wasserstoff, Fluor und Chlor, C₂₋₈-Alkylreste , C₂₋₈-Haloalkylreste, besonders bevorzugt für Fluor, Chlor, Wasserstoff, C₃₋₆-Alkylreste, CF₃ und CF₂H steht.

Beispiele für erfindungsgemäß geeignete fluorierte Carbonäuren sind Trifluoressigsäure, Difluoressigsäure, Difluorchloressigsäure, Chlorfluoressigsäure, 2,3,3,3-Tetrafluorpropionsäure, 2,2,3,3-Tetrafluorpropionsäure, 2,2-Difluorpropionsäure, Pentafluorpropionsäure, 2,3,3,4,4,4-Hexafluorbutancarbonsäure.

### Säurehalogenide (IV)

Die zuvor beschriebenen fluorierten Carbonsäuren werden mit dem Dialkylaminoacrylsäure-Derivat unter Zugabe eines Säurehalogenids der Formel (IV) umgesetzt. 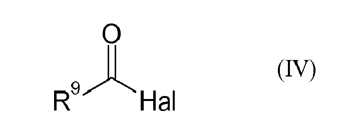 In Formel (IV) ist R⁹ ausgewählt aus C₁₋₁₂-Alkylresten, C₅₋₁₈-Aryl- C₇₋₁₉-Alkylaryl- oder C₇₋₁₉-Arylalkyl- oder Heteroarylresten, vorzugsweise aus C₂₋₈-Alkylresten, besonders bevorzugt aus C₃₋₆-Alkylresten, und Hal ist ausgewählt aus Fluor, Chlor, Brom und Iod, vorzugsweise aus Chlor und Brom, besonders bevorzugt aus Chlor.

Beispiele für erfindungsgemäß geeignete Säurehalogenide sind Essigsäurechlorid, Pivalinsäurechlorid, Isovaleroylchlorid und Benzoylchlorid.

Das molare Verhältnis von Säurehalogeniden der Formel (IV) zur eingesetzten fluorierten Carbonsäure der Formel (II) kann beispielsweise 0,5 bis 5 betragen, bevorzugt 1 bis 3, besonders bevorzugt 2 bis 2,5.

### Basen

Das erfindungsgemäße Verfahren wird in Gegenwart einer Base durchgeführt. Als Basen eignen sich beispielsweise tertiäre Stickstoffbasen, wie z.B, tertiäre Amine, substituierte oder unsubstituierte Pyridine und substituierte oder unsubstituierte Chinoline; Alkali- oder Erdalkalimetallhydroxyde, Hydrogencarbonate oder Carbonate und sonstige anorganische wässrige Basen. Vorzugsweise werden tertiäre Amine, substituierte oder unsubstituierte Pyridine und substituierte oder unsubstituierte Chinoline und solche der allgemeinen Formel (V) eingesetzt,

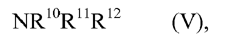

in welcher
- R¹⁰ R¹¹, und R¹²: unabhängig voneinander für C₁₋₁₂-Alkyl, C₆₋₁₈-Aryl, C₇₋₁₉-Alkylaryl- oder C₇₋₁₉-

Arylalkyl stehen können oder jeweils zwei Reste zusammen auch Teil eines 5 bis 8 gliedrigen N-heterocyclischen Restes sein können, oder alle drei Reste zusammen Teil eines N-heterobicyclischen oder N-tricyclischen Restes mit 5 bis 9 Ringatomen pro Cyclus sein können die gegebenenfalls auch andere Heteroatome wie zum Beispiel Sauerstoff enthalten können.

Bevorzugte Beispiele für Basen der allgemeinen Formel (V) sind Triethylamin, Trimethylamin, Diisopropylethylamin, Tri-n-propylamin, Tri-n-butylamin, Tri-n-hexylamin, Tricyclohexylamin, N-Methyl-cyclohexylamin, N-Methylpyrrolidin, N-Methylpiperidin, N-Ethylpiperidin, N,N-Dimethylanilin, N-Methylmorpholin, Pyridin, 2-, 3-, 4-, Picolin, 2-Methyl-5-ethyl-pyridin, 2,6-Lutidin, 2,4,6-Collidin, 4-Dimethylaminopyridin, Chinolin, Chinaldin, N,N,N,N-Tetramethylethyldiamin, N,N-Dimethyl-1,4-diazacyclohexan, N,N-Di-ethyl-1,4-diazacyclohexan, 1,8-Bis-(Dimethylamino)naphthalin, Diazabicylooctan (DABCO), Diazabicyclononan (DBN) und Diazabicycloundecan (DBU). Weiterhin bevorzugt sind Erdalkalimetall- oder Alkalimetallhydroxyde, - carbonate oder- hydrogencarbonate, wie z.B. Natriumhydroxid, Kaliumhydroxid, Lithiumhydroxid, Calciumhydroxid, Natriumcarbonat, Kaliumcarbonat, Natriumhydrogencarbonat und Kaliumhydrogencarbonat eingesetzt. Die anorganische Base kann gegebenenfalls als wässrige Lösung in Konzentrationen zwischen 10 und 40% eingesetzt werden.

Besonders bevorzugt werden Triethylamin, Tributylamin, Pyridin, 2-, 3-, 4-, Picolin, 2-Methyl-5-ethyl-pyridin, 2,6-Lutidin, Natriumhydroxid oder Kaliumhydroxid verwendet.

Das molare Verhältnis von Base zu eingesetzten fluorierten Carbonsäure kann beispielsweise 0,5 bis 10 betragen, bevorzugt 1 bis 8, besonders bevorzugt 1,5 bis 6.

Der Einsatz größerer Mengen an Base ist unkritisch aber unwirtschaftlich.

Bei der Durchführung des erfindungsgemäßen Verfahrens arbeitet man beispielsweise bei Temperaturen von -30 bis 130°C, bevorzugt zwischen 10 und 60°C.

Das erfindungsgemäße Verfahren wird im Allgemeinen unter Normaldruck durchgeführt. Es ist jedoch möglich , unter erhöhtem Druck oder im Vakuum zu arbeiten.

### Lösungsmittel

Die Reaktion kann in Substanz oder in einem Lösungsmittel durchgeführt werden. Vorzugsweise wird die Reaktion in einem Lösungsmittel durchgeführt. Geeignete Lösungsmittel sind beispielsweise ausgewählt aus der Gruppe bestehend aus aliphatischen und aromatischen Kohlenwasserstoffen, wie z.B. n-Hexan, Benzol, Toluol und Xylol, die durch Fluor- und Chloratome substituiert sein können, wie Methylenchlorid, Dichlormethan, Trichlormethan, Tetrachlorkohlenstoff, Fluorbenzol, Chlorbenzol oder Dichlorbenzol; Ethern, wie z.B. Diethylether, Diphenylether Methyl-tert-butylether, Isopropylethylether, Dioxan, Diglym, Dimethylglycol oder THF; Nitrilen wie Methylnitril, Butylnitril oder Phenylnitril; DMF, N,N-Dimethylacetamid wobei Toluol bevorzugt ist.

In einer bevorzugten Ausführungsform des erfindungsgemäßen Verfahrens wird die Base mit der fluorierten Carbonsäure in einem Lösungsmittel oder in Substanz vorgelegt, anschließend wird das Dialkylaminoacrylsäure-Derivat zugegeben und mit dem Carbonsäurechlorid versetzt. Man kann auch die Base mit dem Carbonsäurechlorid in einem Lösungsmittel oder in Substanz vorgelegen, anschließend das Dialkylaminoacrylsäure-Derivat zugeben und mit der fluorierten Carbonsäure versetzen.

Bei einer weiteren erfindungsgemäßen Ausführungsform wird die Base mit der fluorierten Carbonsäure in einem Lösungsmittel oder in Substanz vorgelegt, anschließend wird das Carbonsäurechlorid zugegeben und mit dem Dialkylaminoacrylsäure-Derivat versetzt.

Zur Aufarbeitung kann man beispielsweise so vorgehen, das man gegebenenfalls ausgefallene Salze zum Beispiel durch Filtration, Zentrifugation oder Sedimentation und Dekantieren abtrennt und die so erhaltene Reaktionslösung entweder direkt weiter umsetzt oder zur Gewinnung der 2-Fluoracyl-3-amino-acrylsäure-Derivate, vorzugsweise bis zur Trockene, einengt. Gegebenenfalls können die erhaltenen 2-Fluoracyl-3-amino-acrylsäure-Derivate auch durch Destillation gereinigt werden. Eine weitere Aufarbeitungsmethode kann durch Zugabe von Wasser zum Reaktionsansatzes und anschließender Phasentrennung erfolgen.

Das erfindungsgemäße Verfahren zum Herstellen von 2-Fluoracyl-3-amino-acrylsäure-Derivaten wird in den nachstehenden Beispielen beschrieben, welche die obige Beschreibung weiter illustrieren. Die Beispiele sind jedoch nicht in einschränkender Weise zu interpretieren.

### Herstellungsbeispiele:

### Beispiel 1

Zu einer Lösung von 32,7 g Triethylamin in 60 ml Toluol werden bei Raumtemperatur zuerst 11,6 g Difluoressigsäure und anschließend 14,35 g Dimethylaminoacrylsäureethylester zugesetzt. Zu dieser Lösung werden 28 g Trimethylacetylchlorid zudosiert und 8 h bei Raumtemperatur gerührt. Die Reaktionsmischung wird auf 30 ml Wasser gegossen, die organische Phase wird abgetrennt, die organische Phase wird mit 20 ml Wasser gewaschen, die vereinigten wässrigen Phasen werden nochmals mit 10 ml Toluol extrahiert. Die vereinigten organischen Phasen werden im Vakuum eingeengt. Man erhält 21 g (94% der Theorie) an 2-(Difluoracetyl)-3-(dimethylamino)-acrylsäureethylester.

### Beispiel 2

Zu einer Lösung von 17,8 g Triethylamin in 50 ml Toluol werden bei Raumtemperatur zuerst 5,7 g Trifluoressigsäure und anschließend 7,23 g Dimethylaminoacrylsäureethylester zugesetzt. Zu dieser Lösung werden 14 g Trimethylacetylchlorid zudosiert und 6 h bei Raumtemperatur gerührt. Die Reaktionsmischung wird auf 20 ml Wasser gegossen, die organische Phase wird abgetrennt, die organische Phase wird mit 20 ml Wasser gewaschen, die vereinigten wässrigen Phasen werden nochmals mit 10 ml Toluol extrahiert. Die vereinigten organischen Phasen werden im Vakuum eingeengt. Man erhält 10,7 g (90 % der Theorie) an 2-(Trifluoracetyl)-3-(dimethylamino)-acrylsäureethylester.

### Beispiel 3

Zu einer Lösung von 17,8 g Triethylamin in 50 ml Toluol werden bei Raumtemperatur zuerst 4,8 g Difluoressigsäure und anschließend 7,23 g Dimethylaminoacrylsäureethylester zugesetzt. Zu dieser Lösung werden 16,3 g Benzoylclorid zudosiert und 6 h bei Raumtemperatur gerührt. Die Reaktionsmischung wird abfiltriert, auf 30 ml Wasser gegossen, die organische Phase wird abgetrennt, die organische Phase wird mit 20 ml Wasser gewaschen, die vereinigten wässrigen Phasen werden nochmals mit 10 ml Toluol extrahiert. Die vereinigten organischen Phasen werden im Vakuum eingeengt. Man erhält 2-(Difluoracetyl)-3-(dimethylamino)-acrylsäureethylester in Ausbeute von 81 %.

## Patentansprüche

1. Verfahren zum Herstellen von 2-Fluoracyl-3-amino-acrylsäure-Derivaten der allgemeinen Formel (I) 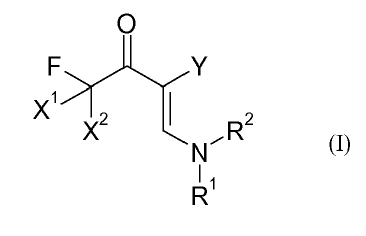 in welcher
R¹ und R² unabhängig voneinander ausgewählt sind aus C₁₋₁₂-Alkylresten, C₅₋₁₈-Aryl-, C₇₋₁₉ Alkylaryl- oder C₇₋₁₉-Arylalkyresten, oder
R¹ und R² zusammen mit dem Stickstoffatom, an das sie gebunden sind, einen 5- bis 6-gliedrigen Ring bilden können, der gegebenenfalls ein oder zwei weitere Hetero-Atome, ausgewählt aus O, S und einer SO₂-Gruppe enthalten kann,
Y ausgewählt ist aus (C=O)OR³, CN und (C=O)NR⁴R⁵, wobei R³ ,R⁴ und R⁵ unabhängig von einander ausgewählt sind aus C₁₋₁₂-Alkylresten, C₅₋₁₈-Aryl-, C₇₋₁₉-Alkylaryl- oder C₇₋₁₉-Arylalkyresten und
X¹ und X² unabhängig voneinander für Fluor, Chlor, Brom, Wasserstoff, C₁₋₁₂-Alkylreste, C₁₋₁₂-Haloalkylreste, C₅₋₁₈-Aryl-, C₇₋₁₉-Alkylaryl- oder C₇₋₁₉-Arylalkyreste stehen,
**dadurch gekennzeichnet, dass** man fluorierte Carbonsäuren der Formel (II) 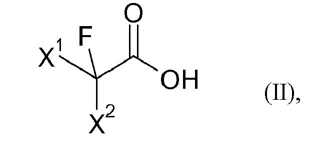 in welcher X¹ und X² die oben genannten Bedeutungen haben, mit 3-Amino-acrylsäure-Derivaten der Formel (III) 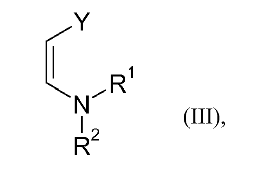 in welcher R¹, R² und Y die oben genannte Bedeutungen haben, in Gegenwart einer Base, mit einem Säurehalogenid der Formel (IV) 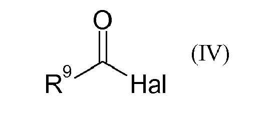 in welcher R⁹ für C₁₋₁₂-Alkylreste, C₃₋₈-Cycloalkylreste, C₁₋₁₂-Haloalkylreste C₅₋₁₈-Aryl- oder C₇₋₁₉-Arylalkyreste und Hal für Halogen steht, umsetzt.

2. Verfahren gemäß Anspruch 1, wobei
R¹ und R² unabhängig voneinander ausgewählt sind aus Methyl, Ethyl, n-Propyl oder iso-Propyl, oder
R¹ und R² zusammen mit dem Stickstoffatom, an das sie gebunden sind, einen Piperidinyl- oder Pyrolidinyl-Ring bilden;
Y ausgewählt ist aus (C=O)OR³, wobei R³ ausgewählt ist aus Methyl, Ethyl, n-Propyl oder iso-Propyl;
X¹ und Wasserstoff; X² unabhängig voneinander ausgewählt sind aus Fluor, Chlor, Brom und
Hal für Chlor oder Brom steht;
R⁹ ausgewählt ist aus C₁₋₁₂-Alkylresten

3. Verfahren gemäß Anspruch 1 oder 2, wobei
R¹ und R² Methyl sind,
Y (C=O)OC₂H₅;
X¹ Fluor ist;
X² Wasserstoff ist;
Hal Chlor ist und
R⁹ Methyl ist.

4. Verfahren gemäß irgendeinem der Ansprüche 1 bis 3, wobei die fluorierte Carbonsäuren ausgewählt sind aus der Gruppe bestehend aus Trifluoressigsäure, Difluoressigsäure, Difluorchloressigsäure, Chlorfluoressigsäure, 2,3,3,3-Tetrafluorpropionsäure, 2,2,3,3-Tetrafluorpropionsäure, 2,2-Difluorpropionsäure, Pentafluorpropionsäure, 2,3,3,4,4,4-Hexafluorbutancarbonsäure.

5. Verfahren gemäß irgendeinem der Ansprüche 1 bis 4, wobei die Dialkylaminoacrylsäure-Derivate ausgewählt sind aus der Gruppe bestehend aus 3-(N,N-Dimethylamino)-acrylsäuremethylester, 3-(N,N-Dimethylamino)-acrylsäureethylester, 3-(N,N-Diethylamino)-acrylsäureethylester, 3-(N,N-Dimethylamino)-acrylsäurenitril, 3-(N,N-Dimethylamino)-acrylsäuredimethylamid und 3-(N,N-Dimethylamino)-acrylsäurediethylamid.

6. Verfahren irgendeinem der Ansprüche 1 bis 5 wobei das molare Verhältnis von Dialkylaminoacrylsäure-Derivaten zu eingesetzten fluorierten Carbonsäure 0,5 bis 3 ist.

7. Verfahren gemäß einem der Ansprüche 1 bis 6, wobei das Säurechlorid ausgewählt ist aus Essigsäurechlorid, Pivalinsäurechlorid, Isovaleroylchlorid und Benzoylchlorid.

8. Verfahren gemäß einem der Ansprüche 1 bis 7, wobei das molare Verhältnis von Säurechlorid zur eingesetzten fluorierten Carbonsäure 0,5 bis 5 ist.

9. Verfahren gemäß einem der Ansprüche 1 bis 8, wobei die Base ausgewählt ist aus Triethylamin, Trimethylamin, Diisopropylethylamin, Tri-n-propylamin, Tri-n-butylamin, Tri-n-hexylamin, Tricyclohexylamin, N-Methyl-cyclohexylamin, N-Methylpyrrolidin, N-Methylpiperidin, N-Ethylpiperidin, N,N-Dimethylanilin, N-Methylmorpholin, Pyridin, 2-, 3-, 4-, Picolin, 2-Methyl-5-ethyl-pyridin, 2,6-Lutidin, 2,4,6-Collidin, 4-Dimethylaminopyridin, Chinolin, Chinaldin, N,N,N,N-Tetramethylethyldiamin, N,N-Dimethyl-1,4-diazacyclohexan, N,N-Di-ethyl-1,4-diazacyclohexan, 1,8-Bis-(Dimethylamino)naphthalin, Diazabicylooctan (DABCO), Diazabicyclononan (DBN) und Diazabicycloundecan (DBU), Natriumhydroxid, Kaliumhydroxid, Lithiumhydroxid, Calciumhydroxid, Natriumcarbonat, Kaliumcarbonat, Natriumhydrogencarbonat und Kaliumhydrogencarbonat.
